(19) Europäisches Patentamt
European Patent Office
Office européen des brevets

(11) EP 1 790 341 A1

(12) EUROPEAN PATENT APPLICATION
published in accordance with Art. 158(3) EPC

(43) Date of publication:
30.05.2007 Bulletin 2007/22

(21) Application number: 05781804.9

(22) Date of filing: 01.09.2005

(51) Int Cl.:
*A61K 31/439* (2006.01) *A61P 25/04* (2006.01)
*A61P 29/02* (2006.01)

(86) International application number:
PCT/CN2005/001384

(87) International publication number:
WO 2006/024236 (09.03.2006 Gazette 2006/10)

(84) Designated Contracting States:
AT BE BG CH CY CZ DE DK EE ES FI FR GB GR HU IE IS IT LI LT LU LV MC NL PL PT RO SE SI SK TR

(30) Priority: 03.09.2004 CN 200410040598

(71) Applicant: Wang, Jiansheng
Sichuan 610072 (CN)

(72) Inventor: Wang, Jiansheng
Sichuan 610072 (CN)

(74) Representative: Hudy, Ludwik et al
Kancelaria Patentowa Patelha
Czernichow 4
32-070 Czernichow, Krakow (PL)

# (54) MEDICINE HAVING ANALGESIC EFFECTS

(57) The pharmaceutical compositions having analgesic effect referred to in this invention are composed of the aconitine ingredients as the active medical ingredients and other acceptable auxiliary ingredients. The active medical ingredients shall at least include the fuziline compound of Formula (I). The drugs exert desirable analgesic effect with low toxicity. They can be made into commonly used oral preparations, injectable preparations and/or external preparations, including ointments, suppositories lotions, medicinal dressings, etc.

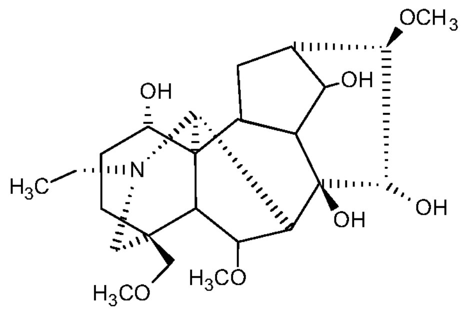

(I)

EP 1 790 341 A1

## Description

Technical Field:

The invention relates to analgesic pharmaceutical compositions, and more specifically, analgesic pharmaceutical compositions prepared from fuziline, an aconitine alkaloid compound, as the active medical ingredient.

Technical Background:

Aconite, the adnation root of Aconitum carmicgaeli Debx, has been used in Traditional Chinese Medicine since ancient times as the medicine of severe heat and a warm agent to revive the yang for resuscitation. Due to its strong toxicity and the closeness between prescribed dose and the poisoning dose, the clinical practice frequently witnesses Examples of poisoning and death. Thus, a great deal of research into its processing, formula, chemistry, toxicity, and pharmacological and clinical effects has been made so as to reduce its toxicity, improve its curative effect, and characterize its active low-toxicity ingredients.

There was a report published in Chinese Traditional and Herbal Medicine 1982: 73 (11), 1-4 by Zhang Dihua that eight ingredients including hypaconitine, mesaconitine and benzoylmesaconitine separated from white sliced aconite proved to be effective in countering inflammation and heart failure.

As for the application of aconitine ingredients to analgesic drugs, there was a report published on Chinese Traditional and Herbal Medicine 2002:33(2), 106-109 by Zhang Yongzhong about researches into the treatment of medium and late stage cancer with the "analgesic soup" made of four traditional Chinese herbs, such as rhizoma corydalis aconite, which proved to be of sound curative effect.

Reports were also made about the extraction, separation, structure and content assessment of single fuziline, such as the report about diterpenes alkaloid fuziline separated from the root tuber of aconitum carmichaeli and its physical and chemical features in Chemical Constituents from Aconitum Carmichaeli published in Natural Product Research and Development (2003:15(4)) by Chen Hongchao. Handbook of Brachylogy Component of Chinese Traditional Drugs pp. 203-204 by Ou Ming also includes the report on medicinal aconite's effects and other ingredients. The active ingredient is aconitine. Other ingredient like hypaconitine and fuziline are also separated. However, a report of the medical effect and toxicity of fuziline as well as the preparation of the mixture of fuziline and other aconite alkaloids has not heretofor been available.

Content of the Invention

Based on the situation described above, this invention relates to an analgesic drug whose active medical ingredient is fuziline, an aconite alkaloid separated from aconite plants, with a sound medical effect and low toxicity. In addition, the invention also relates to an analgesic drug whose active ingredient is the combination of fuziline with one or more than one other aconite ingredients.

The drugs with analgesic effect referred to in this invention are composed of the aconitine ingredients as the active medical ingredients and other acceptable auxiliary ingredients. The active medical ingredients shall at least include the fuziline compound of Formula (I).

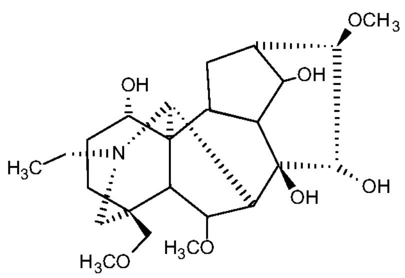

(I)

The active medical ingredients of Formula (I) can be obtained from extraction and separation from medicinal plant aconite according to the methods well documented in the literature.

Besides, the active medical ingredients concerned can be realized through mixing of the above fuziline compound with at least one of such aconite alkaloid ingredients as hypaconitine, mesaconitine and benzoylmesaconitine. Formulas (II), (III) and (IV) show the structures of hypaconitine, mesaconitine and benzoylmesaconitine respectively which can be obtained from extraction and separation from medicinal plant aconite according to the methods well documented in the literature.

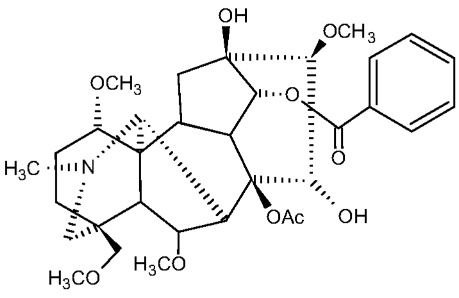

(II)

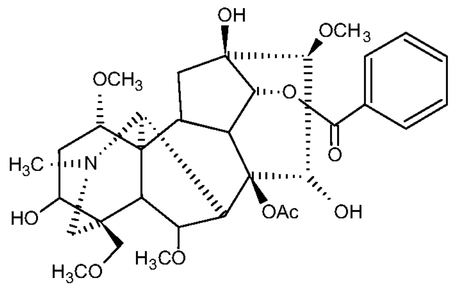

(III)

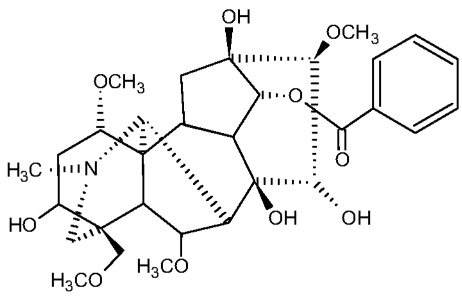

(IV)

The drug mixtures with analgesic effect based on the invention are medicinal preparations made up of drug compounds in the following proportion: 200-600 parts of fuziline, 0-600 parts of benzoylmesaconitine, 0-3 parts of mesaconitine and 0-6 parts of hypaconitine, or 200-600 parts of fuziline, 200-600 parts of benzoylmesaconitine, 1-3 parts of mesaconitine and 2-6 parts of hypaconitine, or to be optimized, or 200 parts of fuziline, 200 parts of benzoylmesaconitine, 3 parts of mesaconitine and 6 parts of hypaconitine, or to be further optimized; or made up of drug compounds in the following proportion: 200-600 parts of fuziline and 1-3 parts of mesaconitine, or 600 parts of fuziline and 1 part of mesaconitine, to be more optimized; or made up of drug compounds in the following proportion: 100-300 parts of fuziline and 1-3 parts of hypaconitine, or 100 parts of fuziline and 3 parts of hypaconitine, to be optimized; or made up of drug compounds in the following proportion: 25-75 parts of fuziline and 25-75 parts of benzoylmesaconitine, or 25 parts of fuziline and 75 parts of benzoylmesaconitine, to be optimized.

The aforementioned medicinal preparations are oral, external and injectable.

The oral preparations include oral liquid, troche, capsule, granule and drop pills; the external preparations include medicinal dressing, ointment, suppository and lotion.

Drugs with analgesic effect can be prepared either as mixtures of fuziline compound as the active medical ingredient or as combinations of fuziline with at least one of hypaconitine, mesaconitine and benzoylmesaconitine as the active medical ingredient collectively, and the applicable, acceptable auxiliary and/or additive ingredients of the drug in the methods of corresponding pharmaceutical preparations. For instance, oral preparations like troche, pill, capsule, granule, drop pill, sustained release formulation, controlled release formulation, etc., can be prepared when blended with such auxiliary materials for oral use as disintegrants, excipients, lubricants, binders, filling materials, etc., through common technological processes. Injectable preparations can be made through combining with appropriate solution applicable to injectable preparation and additive through corresponding technological processes. And external preparations like ointment, suppository lotion, medicinal dressing, etc. can be made by blending with dispersants, condensates, solidifying agents, stabilizers, etc.

The toxicity test of the above active medical ingredients shows that the toxicity of fuziline compound and benzoylmesaconitine is relatively low, without the value of $LD_{50}$ and with the maximum tolerance dose as 1000 mg/kg and that the $LD_{50}$ of mesaconitine is 6.41 mg/kg and the $LD_{50}$ of hypaconitine is 12.8 mg/kg, which proves the safe use of fuziline.

The compatibility of fuziline and other aconite alkaloids is within the safe range of dose. The single use of fuziline acts effectively with low toxicity and high safety. The compatibility of fuziline and the other three aconite alkaloids can bring about synergistic effect. What's more, the drug mixtures and their effect after compatibility is not absolutely in the simple linear relation of dose and effect, i.e., the larger dose, the better effect, but the mixtures have the best effect when administered in a specific proportion of active ingredients. The test results manifest that the pharmaceutical composition in the optimum proportions exerts the best effect with non-linear decline in dose and toxicity. In a word, the drug mixtures of the invention have achieved the goals of significant effect and low toxicity, which provides a new option for the clinical practice.

The following, non-limiting examples, are illustrative to the invention. Any forms of modification, replacement, and alteration based on the idea of the invention are also part of the invention.

Examples:

Example 1: Oral Capsule

The Composition of Capsule One: 80 parts of fuziline and 200 parts of amylose.

The Composition of Capsule Two: 80 parts of fuziline, 0.4 part of mesaconitine and 200 parts of amylose.

Preparation Method: crush and sift the active drug ingredients respectively according to the regular preparation method of capsule; blend them with amylose and an appropriate amount of ethyl alcohol; pelletize and dry them, then blend them with lubricant like magnesium stearate, and fill them up into capsules with required amount. The specification is 0.2 g per capsule.

Example 2: Troche

The Composition of Troche One: 80 parts of fuziline and 200 parts of amylose.

The Composition of Troche Two: 80 parts of fuziline, 1.2 parts of hypaconitine and 200 parts of amylose.

Preparation Method: pelletize, dry and sift according to Example 1. Tablet the compositions with an appropriate amount of talcum powder followed by coating with a coating liquid composed of HPMC, propylene glycol, white titanium pigment, ethyl alcohol, and Tween-80 using a regular coating method. The specification is 0.2 g per tablet. The dose for adult is 1.2 g.

Example 3: Oral Analgesic Granules

The Composition of Granules One: 60 parts of fuziline, 800 parts of amylose and 200 parts of powdered sugar.

The Composition of Granules Two: 80 parts of fuziline, 0.13 part of mesaconitine, 800 parts of amylose and 200 parts of powdered sugar.

Preparation Method: crush and sift the active drug ingredients such as fuziline according to the regular preparation method for granules, then blend them evenly with amylose, powdered sugar and an appropriate amount of ethyl alcohol; pelletize and dry them on the oscillating granulator, and then pack them together in the required amount. The specification is 0.2 g.

Example 4: Oral Analgesic Drop Pills

The Composition: 450 parts of fuziline, 20 parts of carbowax 4000 and appropriate amount of ethyl alcohol.

Preparation Method: according to the regular preparation method for drop pills, dissolve fuziline in ethyl alcohol and melt the carbowax; blend them up and stir them in a high speed stirrer, then drop them into simethicone ($< 5°C$) and make them into drop pills. The specification is 150 mg per pill.

Example 5: Analgesic Injection

The Composition of Injection One: 40 parts of fuziline, 5 parts of Tween-80, 3 parts of EDTA, 1000 parts of water for injection.

The Composition of Injection Two: 5 parts of fuziline, 0.05 part of mesaconitine, 5 parts of Tween-80, 3 parts of EDTA, 1000 parts of water for injection.

Preparation Method: add an appropriate amount of water for injection, Tween-80, EDTA and an appropriate amount of CMC-Na to the active drug ingredient of fuziline; stir them evenly with ultrasonic blending to dissolve them well, then dilute, quantify and filter the solution with water for injection, followed by the capsulation into ampoules. An appropriate amount of hydrochloric acid can be used to adjust the pH to between 4 and 9. The specification is 5 mg per ampoule.

Example 6: Analgesic Emulsion Ointment for External Use

The Composition of Ointment One: 50 parts of fuziline, 100 parts of octadecyl alcohol, 150 parts of stearic acid, 10 parts of sodium dodecyl sulfate, 1 part of ethylparaben and 1,000,000 parts of purified water.

The Composition of Ointment Two: 20 parts of fuziline, 1 part of hypaconitine, 100 parts of octadecyl alcohol, 150 parts of stearic acid, 10 parts of sodium dodecyl sulfate, 1 part of ethylparaben and 1,000,000 parts of purified water.

Preparation Method: heat, dissolve and filter the active drug ingredients such as fuziline with an appropriate amount of ethyl alcohol; blend them with octadecyl alcohol and stearic acid; heat and stir evenly followed by heat preservation as oil phase material ready for use; heat and stir evenly sodium dodecyl sulfate, ethylparaben, and purified water before blended with the oil phase material; then stir them evenly in high speed followed by cooling and capsulation. The content specification is 2%.

Example 7: Analgesic Adhesive Plaster

The Composition of Adhesive Plaster One: 50 parts of fuziline, 400 parts of rubber matrix, 400 parts of colophony, 100 parts of Vaseline, 50 parts of wool fat and 30 parts of zinc oxide.

The Composition of Adhesive Plaster Two: 100 parts of fuziline, 0.05 part of mesaconitine, 1 part of hypaconitine, 400 parts of rubber matrix, 400 parts of colophony, 100 parts of Vaseline, 50 parts of wool fat and 30 parts of zinc oxide.

Preparation Method: according to the regular preparation method for adhesive plaster, carry out the pressing and dipping of the rubber matrix, blend it with the activedrug ingredients such as fuziline, filling material like Vaseline, wool fat, colophony, zinc oxide and disperser followed by plastering and filtration. Apply plaster to the pasting carriers and recycle the solvent followed by packaging with required cutting and lining. The specification is the same as that of Example 5.

Example 8: Analgesic Aerosol

The Composition of Aerosol Medication One: 500 parts of fuziline, 7000 parts of dichlorodifluoromethane, 2500 parts of ethyl alcohol and an appropriate amount of flavoring matter.

The Composition of Aerosol Medication Two: 100 parts of fuziline, 2 parts of benzoylmesaconitine, 7000 parts of dichlorodifluoromethane, 2500 parts of ethyl alcohol and an appropriate amount of flavoring matter.

Preparation Method: add the activedrug ingredients such as fuziline into the ethyl alcohol with an appropriate amount of flavoring matter if needed; evenly blend and filter them before filling into a pressure vessel, then pressurize with dichlorodifluoromethane filtered through micro pores.

Example 9: Analgesic Liniment

The Composition of Liniment One: 400 parts of fuziline, 300 parts of ethyl alcohol and 1000 parts of water.

The Composition of Liniment Two: 400 parts of fuziline, 6 parts of mesaconitine, 10 parts of hypaconitine, 300 parts of benzoylmesaconitine, 300 parts of ethyl alcohol and 1000 parts of water.

Preparation Method: add the active drug ingredients such as fuziline into the ethyl alcohol; evenly blend and filter it; adjust to the gross amount with distilled water.

Example 10: Analgesic Lotion

The Composition of Lotion One: 400 parts of fuziline, 100 parts of ethyl alcohol and 1000 parts of water.

The Composition of Lotion Two: 400 parts of fuziline, 90 parts of mesaconitine, 80 parts of hypaconitine, 300 parts of benzoylmesaconitine, 100 parts of ethyl alcohol and 1000 parts of water.

Preparation Method: the same as in Example 9.

Example 11: Analgesic Pellicle

The Composition of Pellicle One: 400 parts of fuziline, 30 parts of polyvinyl alcohol, 100 parts of glycerol and 50 parts of Tween-80.

The Composition of Pellicle Two: 400 parts of fuziline, 90 parts of mesaconitine, 300 parts of benzoylmesaconitine, 30 parts of polyvinyl alcohol, 100 parts of glycerol and 50 parts of Tween-80.

Preparation Method: crush the active drug ingredients such as fuziline; blend evenly with glycerol and Tween-80 before blending with dilluent made of polyvinyl alcohol, form with an appropriate amount of liquid paraffin into pellicle; ready for use after drying.

Example 12: Analgesic Moist Dressing

The Composition of Moist Dressing One: 140 parts of fuziline, 150 parts of ethyl alcohol and 1000 parts of purified water.

The Composition of Moist Dressing Two: 150 parts of fuziline, 6 parts of hypaconitine, 100 parts of benzoylmesaconitine, 150 parts of ethyl alcohol and 1000 parts of purified water.

Preparation Method: dissolve the active drug ingredients such as fuziline in ethyl alcohol, then dilute it with water; dip richly the hospital gauze into solution; take out the gauze blended with solution, followed by packaging.

Test 1 Acute Toxicity Test of Fuziline

The test shows: The maximum tolerance dose of single stomach perfusion of fuziline for the Kunming breed mice is 1 g/kg.

Test of the maximum tolerance dose

1. Test Materials

1.1 Test Drug

Pharmaceutical fuziline, crystal granule, supplied by Chengdu Zhizhi Pharmaceuticals Co., Ltd

1.2 Test Animal

Kunming breed mice with equal number of each gender, weighing 18-22 g, supplied by Experimental Animal Center of Sichuan University, first class animal; certificate: Sichuan Animal Administration, Quality No. 67; free intake of water; breeding house temperature: 21±2°C; comparative humidity:50-60%.

2. Test Method

Make ultrasound-assisted suspension the crystal granule of pharmaceutical fuziline with distilled water with the concentration of 0.5 g/ml (containing 1% of carboxymethylcellulose sodium); perfuse the stomach of the 20 Kunming

breed mice that have the access to water but no food for 14 hours with 0.2 ml/10g of fuziline suspension; then observe the animals immediately for 7 days consecutively and record their behavior and conditions of skin and hair luster, body weight and death.

3. Test Result

During 15 minutes to 4 hours, most mice crouch with and piloerection little movement; after 4 hours, their behavior, movement and diet returned gradually to normal. During the observation period of 7 days, there is no death of animals observed. Their diet and movement behavior is normal, hair color glossy and weight gaining normal (the body weight before test is 18.9±0.7 g, whereas the body weight after the test is 25.4±2.0 g). 7 days later, the organs of mice autopsied show no significant anomalies through visual inspection. Therefore, the maximum tolerance dose of single stomach perfusion of fuziline for the Kunming breed mice is 1 g/kg.

4. Conclusion

The maximum tolerance dose of single stomach perfusion of fuziline for the Kunming breed mice is 1 g/kg.

Test 2 Acute Toxicology Test of the Mixture of Fuziline and Benzoylmesaconitine

The test shows: The $LD_{50}$ and 95% of the confidence interval of single stomach perfusion of fuziline and benzoylmesaconitine for the Kunming breed mice is 1.384 g/kg and 1.192-1.605 g/kg respectively.

Test of the half-number lethal dose ($LD_{50}$)

1. Test Materials

1.1 Test Drug

Benzoylmesaconitine, fuziline, light yellow powder, supplied by Chengdu Zhizhi Pharmaceuticals Co., Ltd.

Dehydrated alcohol, analytically pure, supplied by Chengdu Kelong Chemical Reagent Factory; batch number: 20040803.

Add 2.5 g of benzoylmesaconitine and fuziline each into 4 ml of dehydrated alcohol, followed by ultrasound-assisted dissolution with 42°C water bath; add distilled water up to 40 ml to compound 125 mg/ml light yellow solution, then dilute it into solution of 93.7, 70.3, 52.7, 39.6 mg/ml in the proportion of 1:0.75.

Then blend 1 ml of dehydrated alcohol and 10 ml of distilled water to compound 10% alcohol.

1.2 Test Animal

Kunming breed mice with equal number of each gender, weighing 18-22 g, supplied by Experimental Animal Center of Sichuan University, first class animal; certificate: Sichuan Animal Administration, Quality No. 67; free intake of water; breeding house temperature: 21±2°C; comparative humidity: 50-60%.

2. Test Method

Ascertain the maximum lethal dose as 2.5 g/kg and the minimum lethal dose as 0.5 g/kg through preliminary tests. Divide randomly the 120 Kunming breed mice with the access to water but no food for 14 hours into 6 groups with equal number of each gender. Beginning from 2.5 g/kg with the declining proportion of 1:0.75, perfuse the stomach of mice of the 5 groups with 0.2 ml/10g of fuziline and benzoylmesaconitine respectively and with a replacement of 10% alcohol for the negative control group; then observe the animals immediately for 14 days consecutively and record their toxic reaction and death.

3. Test Results

No apparent reaction takes place on the negative control group administered by 10% alcohol. During 10 minutes to 4 hours, with the increase of dose, the mice of the administration group (with 50% of fuziline and each) show an increasing degree of crouching, less movement, piloerection, pronation, asphyxia twitch. As the dose increases, more mice die gradually. Autopsied mice show no significant anomaly of the organs through visual inspection. Survived mice return to normal one day later in terms of behavior, movement, diet, hair color and weight gaining. After 14 days, no apparent disparity of weight is shown among each group. The organs of mice autopsied show no significant anomaly through visual inspection. Table 1 shows mortality statistics in the different dose groups.

Table 1. Mortality statistics of the different dose groups.

| Group | Dose (g/kg) | Logarithm of dose | Animal number | Death number | Death rate (%) |
|---|---|---|---|---|---|
| 1 | 2.50 | 0.398 | 20 | 20 | 100 |
| 2 | 1.875 | 0.273 | 20 | 14 | 70 |
| 3 | 1.406 | 0.148 | 20 | 11 | 55 |
| 4 | 1.055 | 0.023 | 20 | 5 | 25 |
| 5 | 0.796 | -0.099 | 20 | 1 | 5 |
| Negative control | - | - | 20 | 0 | 0 |

The death rate of each dose calculated by the Bliss method using NDST software indicates that the $LD_{50}$ of drugs with 50% of fuziline and benzoylmesaconitine each is 1.384 g/kg and 95% of the confidence interval is 1.192-1.605 g/kg.

4. Conclusion

The toxicity of fuziline and benzoylmesaconitine is comparatively low. The $LD_{50}$ and 95% of the confidence interval of single stomach perfusion for the Kunming breed mice are 1.384 g/kg and 1.192-1.605 g/kg, respectively. According to the animal reaction after administration, the possibly injured target organs include the central nervous system, the autonomic nervous system, the respiratory system, etc.

The proportionate mixture of fuziline and benzoylmesaconitine, within the $LD_{50}$ measured from the previous test and the maximum tolerance dose, can reach the goal of high effect and low toxicity.

Test 3 Pharmacodynamic Tests of Fuziline, Hypaconitine, Mesaconitine and Benzoylmesaconitine

1. Analgesic test of body twisting of mice

Test animals: Kunming breed mice with equal number of each gender, weighing 18-22 g, fed in the same condition.

Test drug: solution of fuziline with the content of 20, 10, 5 and 2.5 mg/ml each with distilled water.

Contra positive A: 2 mg/ml solution of the pethidine hydrochloride injection (supplied by Qinghai Pharmaceuticals Factory Co., Ltd, batch No.: 20020510) with the normal saline.

Contra positive B: 10 mg/ml solution of the grinded enteric coated aspirin tablet (supplied by Nanjing Baijingyu Pharmaceutical Co., Ltd., batch No.: 031016) with distilled water.

Divide the mice randomly into groups of 10, with one blank control group; perfuse the stomach of mice with distilled water by the dose of 0.2 ml/10g; inject into the stomach cavity of mice of the positive control group A with contra positive A by the dose of 40 mg/kg (20 times the practice dose); perfuse the stomach of mice of the positive control group B with contra positive B by the dose of 200 mg/kg (20 times the practice dose); perfuse the stomach of mice of other groups with fuziline by different dose gradients; the administration volume for each group is 0.2 ml/10g; except mice of the group with the contra positive A are injected into the stomach cavity with 0.2 ml of 0.6% glacial acetic acid (analytically pure, 99.5%, supplied by) 15 minutes after administration, the mice of all other groups are injected into the stomach cavity with 0.2 ml of 0.6% glacial acetic acid 30 minutes after administration; observe the number of body twisting of each mouse 5 to 20 minutes after the injection; make statistical analysis of the data to calculate the increasing rate of pain threshold and the standard deviation of each group; compare the intergroup differences between each drug group and the negative control group with t-test. The test results are illustrated in Table 2 as follows.

Table 2. Result of analgesic test of body twisting of mice

| Group | Dose (mg/kg) | Body twisting number ($\bar{\chi} \pm$ SD) | Pain suppression rate (%) |
|---|---|---|---|
| Blank control | -- | 34.3±8.0 | -- |
| Pethidine hydrochloride | 40 | 0±0*** | 100.00 |
| Aspirin | 200 | 3.1±3.3*** | 90.96 |
| Fuziline | 400 | 13.0±10.2*** | 62.10 |
| | 200 | 14.7±10.8** | 57.14 |
| | 100 | 23.6±7.9** | 31.20 |
| | 50 | 29.5±14.6 | 13.99 |
| Note: compared with the blank control group, *P<0.05, **P<0.01, ***P< 0.001 | | | |

The injection of acetic acid causes the mice lasting pain with reactions of frequent hindleg stretching, rump rising (body twisting). The result of Table 1 shows the two contra positives, pethidine hydrochloride and aspirin have remarkably reduced the number of body twisting caused by acetic acid (p<0.001), with remarkable analgesic effect and the pain suppression rate of 100% and 90.96%. The mixtures at the doses of 400 mg/kg and 200 mg/kg of fuziline show a varying degree of declining numbers of body twisting caused by acetic acid, with obvious analgesic effect and certain dose dependence, as well.

Result of similar tests of hypaconitine, mesaconitine and benzoylmesaconitine are shown in Table 3, as follows:

Table 3. Result of analgesic test of body twisting of mice

| Group | Dose (mg/kg) | Body twisting number ($\bar{\chi} \pm$ SD) | Pain suppression rate (%) |
|---|---|---|---|
| Blank control | - | 39.3±13.8 | - |
| Pethidine hydrochloride | 40 | 0±0*** | 100.00 |
| Aspirin | 200 | 3.1±3.2*** | 92.11 |
| Hypaconitine | 4 | 2.8±4.5*** | 92.88 |
| | 2 | 23.6±10.9* | 39.95 |
| | 1 | 24.5±9.8* | 37.66 |
| | 0.5 | 27.6±11.5 | 29.77 |
| Mesaconitine | 2 | 19.3±10.0△△ | 50.89 |
| | 1 | 21.8±12.6△△ | 44.53 |
| | 0.5 | 20.0±9.6△△ | 49.11 |
| | 0.25 | 33.6±11.5 | 14.50 |
| Benzoylmesaconitine | 400 | 20.4±12.9▲▲ | 40.52 |
| | 200 | 21.0±15.1▲ | 38.78 |
| | 100 | 27.4±15.9 | 20.12 |
| | 50 | 27.2±12.2 | 20.70 |
| Note: compared with the blank control group, *P<0.05, **P<0.01, ***P< 0.001; △P<0.01; ▲P<0.05, ▲▲P<0.01. | | | |

The result of Table 3 shows the remarkable analgesic effect of contra positives, pethidine hydrochloride and aspirin. The pain suppression rate is 100% and 92.11% respectively. All does groups of hypaconitine, except the 0.5 mg/kg dose group, show obvious analgesic effect with certain dose dependence. All does groups of mesaconitine, except the 0.25 mg/kg dose group, show obvious analgesic effect with certain dose dependence. All does groups of hypaconitine, except the 400 mg/kg and 200 mg/kg dose groups, show obvious analgesic effect with certain dose dependence as well.

2. Hot-plate Test

The division of animal groups, content of test drugs, dose and administration is the same as those in the body twisting test, while the contra positive is pethidine hydrochloride only.

Keep the temperature of aqueous bath as 55±0.5°C; put the mice into the aluminum bucket of constant temperature; record the time length (pain threshold value) between their being put in and the hindleg sucking; record twice with an interval of 5 minutes; take the average value as the pain threshold value before administration; divide mice whose pain threshold value is between 5 and 30 seconds into groups with 10 each; measure twice the pain threshold values of each group after the administration of 15, 30, 60, 90, 120 minutes and the average values as well as its increasing rate during each time phase; make statistical analysis of the data to calculate the increasing rate of pain threshold and the standard deviation of each group; compare the intergroup differences between each drug group and the negative control group with t-test.

In the course of the test, the mice show up reactions of hindleg sucking, hindleg kicking, skipping, etc; take the reaction of hindleg sucking as the pain indicator. The test results are shown in Table 4, as follows.

Table 4. Results of hot-plate analgesic test of mice

| Group | Dose (mg/ kg) | Increasing rate of threshold pain after administration(%)($\bar{x}$±SD) | | | | |
|---|---|---|---|---|---|---|
| | | 15 minutes | 30 minutes | 60 minutes | 90 minutes | 120 minutes |
| Blank control | - | 24.5 ±51.5 | -12.2 ±36.1 | 2.4 ±43.4 | 15.6 ±51.7 | 26.9 ±51.2 |
| Pethidine hydrochloride | 40 | 208.7 ±59.8** | 164.8 ±63.6*** | 108.4 ±90.4** | 11.8 ±39.3 | 42.2 ±66.1 |

(continued)

| Group | Dose (mg/ kg) | Increasing rate of threshold pain after administration(%)($\bar{x}\pm$SD) | | | | |
|---|---|---|---|---|---|---|
| | | 15 minutes | 30 minutes | 60 minutes | 90 minutes | 120 minutes |
| fuziline | 400 | 162.9 ±176.1 | 146.8 ±122.5*** | 59.5 ±93.8 | 32.7 ±63.5 | 48.5 ±76.4 |
| | 200 | 83.5 ±105.7 | 32.3 ±58.3 | 64.6 ±80.3 | 34.4 ±49.7 | 100.8 ±79.8* |
| | 100 | 10.7 ±30.9 | 65.2 ±83.2* | 34.6 ±54.8 | 29.4 ±53.2 | 38.3 ±67.0 |
| Note: compared with the blank control group, *P<0.05, **P<0.01, ***P<0.001 | | | | | | |

The result of Table 4 shows remarkable rise of the increasing rate of threshold pain in the large dose groups of fuziline drug after the administration of 15 and 30 minutes, the medium dose groups of drug fuziline after the administration of 120 minutes, and the small dose groups of drug fuziline after the administration of 30 minutes.

The test result of hypaconitine, mesaconitine and benzoylmesaconitine is as Table 5 shows.

Table 5. Result of hot-plate analgesic test of mice

| Group | Dose (mg/ kg) | Increasing rate of threshold pain after administration (%)($\bar{x}\pm$SD) | | | | |
|---|---|---|---|---|---|---|
| | | 15 minutes | 30 minutes | 60 minutes | 90 minutes | 120 minutes |
| Blank control | - | 12.1 ±45.0 | -6.3 ±31.4 | -0.5 ±38.7 | -11.3 ±34.2 | 11.1 ±38.8 |
| Contra positive A | 40 | 284.4 ±229.5* | 181.3 ±98.4*** | 118.9 ±117.5** | 44.7 ±64.4* | 0.7 ±48.3 |
| Hypaconitine | 4 | 142.7 ±139.4* | 104.1 ±137.4* | 81.7 ±100.0* | 34.4 ±59.3* | 59.1 ±55.6* |
| | 2 | 72.4 ±57.6* | 36.0 ±53.4 | 17.3 ±56.1 | 15.3 ±60.1 | -13.0 ±33.1 |
| | 1 | -16.6 ±31.9 | -31.9 ±24.2 | -18.1 ±34.5 | -13.0 ±14.9 | 28.3 ±56.3 |
| Mesaconitine | 100 | 124.8 ±165.4 | 97.7 ±184.6 | 64.3 ±149.5 | 108.3 ±105.0△△ | 49.0 ±52.9 |
| | 50 | 21.4 ±42.9 | 14.4 ±34.7 | 7.9 ±37.8 | 30.4 ±44.1△ | 36.0 ±42.6 |
| | 25 | 39.7 ±76.6 | 59.2 ±146.9 | 37.2 ±88.5 | 61.3 ±82.1△ | 80.7 ±100.1 |
| | 12.5 | 12.1 ±45.0 | -6.3 ±31.4 | -0.5 ±38.7 | -11.3 ±34.2 | 11.1 ±38.8 |
| Benzoylmes aconitine | 400 | 41.3 ±90.2 | 35.2 ±57.2▲ | 78.8 ±104.0▲ | 59.5 ±88.5 | 53.7 ±117.0 |
| | 200 | 22.1 ±52.0 | 32.9 ±45.1▲ | 40.3 ±76.4 | 46.0 ±87.4 | 53.6 ±93.9 |
| | 100 | -0.4 ±33.8 | 13.7 ±52.3 | 64.6 ±76.7▲ | 71.1 ±104.5 | 106.2 ±116.5 |
| Note: compared with the blank control group, *P<0.05, **P<0.01, ***P< 0.001; △P<0.05, △△P<0.01; ▲P<0.05, ▲▲P<0.01. | | | | | | |

The results in Table 5 shows remarkable rise of the pain threshold in the large dose groups of hypaconitine drug at each time phase, whose analgesic effect lasts longer that the groups of contra positive A, with outstanding analgesic effect within 2 hours.

Test 4. Analgesic Effects of the Mixtures in Different Proportions of Fuziline, Mesaconitine and Benzoylmesaconitine with Hypaconitine

Test result shows compound medicines composed of the mixtures in different proportions of fuziline, mesaconitine and benzoylmesaconitine with hypaconitine have remarkable analgesiceffect. The effect of the mixture in the proportion of 200:200:3:6 is the strongest when the mixtures with proportions of fuziline, mesaconitine and benzoylmesaconitine with hypaconitine are 200:200:1:2, 600:600:1:2, 200:600:3:2, 600:200:1:6 and 200:200:3:6. Therefore, the optimum mixture proportion is 200:200:3:6.

I. Test Materials

1. Test Drug and Preparation

Benzoylmesaconitine, light yellow powder; fuziline and mesaconitine, near-white power; hypaconitine, white crystal granule, supplied by Chengdu Zhizhi Pharmaceuticals Co., Ltd; prepare 250 mg of fuziline and benzoylmesaconitine each before mixing with 2.5 ml of dehydrated alcohol, followed by ultrasound-assisted dissolution with 42°C water bath; add distilled water up to 25 ml to compound 10 mg/ml of solution composed of fuziline and benzoylmesaconitine each; prepare 1.25 mg of mesaconitine before mixing with 2.5 ml of dehydrated alcohol, followed by ultrasound-assisted dissolution with 42°C water bath into 0.05 mg/ml of solution composed of mesaconitine; prepare 2.5 mg of hypaconitine before mixing with 2.5 ml of dehydrated alcohol, followed by ultrasound-assisted dissolution with 42°C water bath into 0.1 mg/ml of solution composed of hypaconitine; prepare solution of different volumes of fuziline, benzoylmesaconitine, mesaconitine and hypaconitine into solution in different proportions of fuziline, benzoylmesaconitine, mesaconitine and hypaconitine according to Table 6.

Table 6. Mixtures in different proportions of fuziline, benzoylmesaconitine, mesaconitine and hypaconitine

| Group | Sample volume (ml) | | | | Dose (mg/kg) | | | | Proportion |
|---|---|---|---|---|---|---|---|---|---|
| | Fuziline | Benzoylmesaconitine | Mesaconitine | Hypaconitine | Fuziline | Benzoylmesaconite | Mesaconitine | Hypaconitine | |
| ① | 1.25 | 1.25 | 1.25 | 1.25 | 25 | 25 | 0.125 | 0.25 | 200:200:1:2 |
| ② | 1.88 | 1.88 | 0.62 | 0.62 | 37.5 | 37.5 | 0.062 | 0.125 | 600:600:1:2 |
| ③ | 0.62 | 1.88 | 1.88 | 0.62 | 12.5 | 37.5 | 0.188 | 0.125 | 200:600:3:2 |
| ④ | 1.88 | 0.62 | 0.62 | 1.88 | 37.5 | 12.5 | 0.062 | 0.375 | 600:200:1:6 |
| ⑤ | 0.62 | 0.62 | 1.88 | 1.88 | 12.5 | 12.5 | 0.188 | 0.375 | 200:200:3:6 |

Enteric coated aspirin tablet, white, 25 mg/pill, supplied by Xuzhou Enhua Pharmaceuticals Co., Ltd, batch No.:20050312; take 4 pills of enteric coated aspirin tablet; grind and add with distilled water up to 10 ml to compound 10 mg/ml of solution composed of aspirin;

Then blend 1 ml of dehydrated alcohol and 10 ml of distilled water to compound 10% alcohol.

2. Test Animal

Kunming breed mice with equal number of each gender, weighing 18-22 g, supplied by Experimental Animal Center of Sichuan University, first class animal; certificate: Sichuan Animal Administration, Quality No. 10; free intake of water; breeding house temperature: $21\pm2°C$; comparative humidity:50-60%.

3. Reagents

Glacial acetic acid, analytically pure, Chengdu Chemical Reagent Factory, batch No.: 20050423.

Dehydrated alcohol, analytically pure, Chengdu Kelong Chemical Reagent Factory, batch No.:20050313.

II. Test Method

Divide at random the 70 Kunming breed mice, weighing 18-22 g, into 7 groups with equal number of each gender and each group, with the first group as the negative control group and the second group as the positive control group; perfuse the stomach of the first group mice with 0.2 ml/10g of 10% alcohol; perfuse the stomach of the second group mice with 200 mg/kg (20 times the practice dose) of aspirin; the $3^{rd}$, $4^{th}$, $5^{th}$, $6^{th}$ and $7^{th}$ group are groups in five different proportions of fuziline, benzoylmesaconitine, mesaconitine and hypaconitine; perfuse the groups with solution in five different proportions of fuziline, benzoylmesaconitine, mesaconitine and hypaconitine; the administration volume is 0.2 ml/10g; inject into the stomach cavity of the mice with 0.2 ml of 0.6% glacial acetic acid 30 minutes after administration; observe the number of body twisting of each mouse 5 to 10 minutes after the injection of acetic acid; make statistical analysis of the data to calculate the increasing rate of pain threshold and the standard deviation of each group; compare the intergroup differences between each drug group and the negative control group with t-test.

$$\text{Pain suppression rate (\%)} = \frac{\text{Body twisting number of blank group} - \text{Body twisting number of group administered drugs}}{\text{Body twisting number of blank group}} \times 100\%$$

III. Test Result

The injection of acetic acid causes the mice lasting pain with reactions of frequent hindleg stretching, rump rising (body twisting). The number of body twisting caused by acetic acid in the positive control group of aspirin is declining remarkably ($p<0.001$), with remarkable analgesic effect and the pain suppression rate of 88.0%. The compound medicines composed of the mixtures of fuziline, benzoylmesaconitine and mesaconitine with hypaconitine in the proportions of 200:200:1:2, 600:600:1:2, 200:600:3:2, 600:200:1:6 and 200:200:3:6 can ease the reactions of mice caused by acetic acid to various degrees. The number of body twisting is quite from that of the negative control group with remarkable analgesic effect and the pain suppression rate of 48.9%, 43.8%, 60.2%, 54.5% and 64.2%. The test result is as Table 7 shows. In terms of the pain suppression rate, the analgesic effect of the mixture in the proportion of 200:200:3:6 is the strongest. Therefore, the optimum mixture proportion is 200:200:3:6.

Table 7 Effects of the mixture of fuziline benzoylmesaconitine, mesaconitine and hypaconitine in different proportions on the pain reaction of mice caused by acetic acid

| Group | Dose (mg/kg) | | | | Number of animals | Body twisting number ($\bar{x}\pm$SD) | Pain suppression rate (%) |
|---|---|---|---|---|---|---|---|
| | Fuziline | Benzoyl mesaconitine | Mesaconitine | Hypaconitine | | | |
| Negative control group | -- | -- | -- | -- | 10 | 27.4±10.1 | -- |
| Aspirin | -- | -- | -- | -- | 10 | 3.3±3.2*** | 88.0 |
| ① | 25 | 25 | 0.125 | 0.25 | 10 | 14.0±9.4** | 48.9 |
| ② | 37.5 | 37.5 | 0.062 | 0.125 | 10 | 15.4±6.7** | 43.8 |
| ③ | 12.5 | 37.5 | 0.188 | 0.125 | 10 | 10.9±8.0*** | 60.2 |
| ④ | 37.5 | 12.5 | 0.062 | 0.375 | 10 | 12.5±7.2** | 54.4 |
| ⑤ | 12.5 | 12.5 | 0.188 | 0.375 | 10 | 9.8±6.4*** | 64.2 |
| Note: t-test, compared with that of the negative control group, *p<0.05, **p<0.01, ***p<0.001 | | | | | | | |

IV. Conclusion

Test result shows compound medicines composed of the mixtures in different proportions between pharmaceutical fuziline, mesaconitine, benzoylmesaconitine and hypaconitine have remarkable analgesic effect. In terms of the pain suppression rate, the dose and medical effect of compound medicines composed of the mixtures with proportions at 200:200:1:2, 600:600:1:2, 200:600:3:2, 600:200:1:6 and 200:200:3:6 have no dose dependence, with the analgesic effect of the proportion of 200:200:3:6 as the strongest. The proportion has the lowest dose and strongest effect, reaching the goal of high effect and low toxicity.

Test 5. Analgesic Effect of the Mixtures of Fuziline and Mesaconitine in Different Proportions

Test result shows that fuziline at the dose of 100 kg/kg and mesaconitine at the dose of 0.5 mg/kg have remarkable analgesic effects, with the pain suppression rate of 31.3% and 62.6%, respectively. The compound medicines composed of the mixtures of fuziline and mesaconitine in the proportion of 600:1, 200:1 and 200:3 are also of remarkable effect of easing the body twisting reaction caused by the acetic acid, with the pain suppression rate of 86.0%, 82.1% and 65.4% respectively, higher than those of the 100 kg/kg of fuziline and 0.5 mg/kg of mesaconitine. In terms of the pain suppression rate, the larger proportion of fuziline, the greater analgesic effect. The proportion of the mixture of fuziline and mesaconitine as 600:1 has the strongest analgesic effect among the 3 different proportions.

I. Test Materials

1. Test Drug and Preparation

Fuziline, benzoylmesaconitine, near-white powder, supplied by Chengdu Zhizhi Pharmaceuticals Co., Ltd. Prepare 250mg of fuziline before mixing with 2.5 ml of dehydrated alcohol, followed by ultrasound-assisted dissolution with 42°C water bath; add distilled water up to 25 ml to compound 10 mg/ml of solution composed of fuziline; prepare 1.25 mg/ml of mesaconitine before mixing with 2.5 ml of dehydrated alcohol, followed by ultrasound-assisted dissolution with 42°C water bath; add distilled water up to 25 ml to compound 0.05 mg/ml of solution composed of mesaconitine; prepare solution of different volumes of fuziline and solution of different volumes of mesaconitine into solution in different proportions of fuziline and mesaconitine according to Table 8.

Enteric coated aspirin tablet, white, 25 mg/pill, supplied by Xuzhou Enhua Pharmaceuticals Co., Ltd, batch No.: 20050312; Take 4 pills of enteric coated aspirin tablet; grind and add with distilled water up to 10 ml to compound 10 mg/ml of solution composed of aspirin.

Then blend 1 ml of dehydrated alcohol and 10 ml of distilled water to compound 10% alcohol.

Table 8 mixtures of fuziline and mesaconitine in different proportions

| Group | Sampling dose (ml) | | | Dose (mg/kg) | | Proportion |
|---|---|---|---|---|---|---|
| | Fuziline | Mesaconitine | Distilled water | fuziline | mesaconitine | |
| ① | 2.5 | - | 2.5 | 100 | - | - |
| ② | - | 2.5 | 2.5 | - | 0.5 | - |
| ③ | 3.75 | 1.25 | - | 150 | 0.25 | 600:1 |
| ④ | 2.5 | 2.5 | - | 100 | 0.5 | 200:1 |
| ⑤ | 1.25 | 3.75 | - | 50 | 0.75 | 200:3 |

2. Test Animal

Kunming breed mice with equal number of each gender, weighing 18-22 g, supplied by Experimental Animal Center of Sichuan University, first class animal; certificate: Sichuan Animal Administration, Quality No. 10; free intake of water; breeding house temperature: 21±2°C; comparative humidity: 50-60%.

3. Drugs

Glacial acetic acid, analytically pure, Chengdu Chemical Reagent Factory, batch No.: 20050423.

Dehydrated alcohol, analytically pure, Chengdu Kelong Chemical Reagent Factory, batch No.: 20050313.

II. Test Method

Divide randomly the 70 Kunming breed mice, weighing 18-22 g, into 7 groups with equal number of each gender and each group, with the first group as the negative control group and the second group as the positive control group; perfuse the stomach of the first group mice with 0.2 ml/10g of 10% alcohol; perfuse the stomach of the second group mice with 200 mg/kg (20 times the practice dose) of aspirin; the 3$^{rd}$, 4$^{th}$, 5$^{th}$, 6$^{th}$ and 7$^{th}$ group are of fuziline, mesaconitine and their three different proportions; perfuse the groups with solution with fuziline, mesaconitine and their three different proportions; the administration volume is 0.2 ml/10g; inject into the stomach cavity of the mice with 0.2 ml of 0.6% glacial acetic acid 30 minutes after administration; observe the number of body twisting of each mouse 5 to 10 minutes after the injection; make statistical analysis of the data to calculate the increasing rate of pain threshold and the standard deviation of each group; compare the intergroup differences between each drug group and the negative control group with t-test.

$$\text{Pain suppression rate (\%)} = \frac{\text{Body twisting number of blank group} - \text{Body twisting number of group administered drugs}}{\text{Body twisting number of blank group}} \times 100\%$$

III. Test Results

The injection of acetic acid causes the mice lasting pain with reactions of frequent hindleg stretching, rump rising (body twisting). The number of body twisting caused by acetic acid in the positive control group of aspirin is declining remarkably (p<0.001), with remarkable analgesic effect and the pain suppression rate of 82.7%. The mixtures at the doses of 100 mg/kg of fuziline and 0.5 mg/kg of mesaconitine can remarkably ease the reaction of body twisting caused by acetic acid, with the pain suppression rate of 31.3% and 62.6% respectively. The mixtures with proportions of fuziline and mesaconitine as 600:1, 200:1 and 200:3 can also ease the body twisting reaction to various degrees. The number of body twisting is quite from that of the negative control group with remarkable analgesic effect and the pain suppression rate of 86.0%, 82.1% and 65.4%. The test result is as Table 9 shows. In terms of the pain suppression rate, the analgesic effect of the mixture of fuziline and mesaconitine in the proportion of 600:1 is the strongest. Therefore, the optimum proportion of the mixture of fuziline and mesaconitine is 600:1. The test also shows though the mixture of fuziline at the dose of 100 mg/kg may not have a analgesic effect as strong as that of mesaconitine at the dose of 0.5 mg/kg, with the increase of the proportion of fuziline, its analgesic effect increases.

Table 9 Effects of the mixture of fuziline and mesaconitine in different proportions on the Pain reaction of mice caused by acetic acid

| Group | Dose (mg/kg) | | Number of animals | Body twisting number ($\bar{x}\pm$SD) | Pain suppression rate(%) |
|---|---|---|---|---|---|
| | Fuziline | Mesaconitine | | | |
| Negative control | -- | -- | 10 | 17.9±6.0 | -- |
| Aspirin | -- | -- | 10 | 3.0±3.3*** | 82.7 |
| ① | 100 | -- | 10 | 12.3±5.8* | 31.3 |
| ② | -- | 0.5 | 10 | 6.7±7.6** | 62.6 |
| ③ | 150 | 0.25 | 10 | 2.5±2.2*** | 86.0 |
| ④ | 100 | 0.5 | 10 | 3.2±4.5*** | 82.1 |
| ⑤ | 50 | 0.75 | 10 | 6.2±10.0** | 65.4 |
| Note: t-test, compared with that of the negative control group, *p<0.05**, p<0.01, ***p<0.001 | | | | | |

IV. Test Method

Conclusions

The mixtures at the doses of 100 mg/kg of fuziline and 0.5 mg/kg of mesaconitine have outstanding analgesic effects, with the pain suppression rate of 31.3% and 62.6%, respectively. The mixtures with proportions of fuziline and mesaconitine as 600:1, 200:1 and 200:3 can also ease the body twisting reaction remarkably, with the pain suppression rate of 86.0%, 82.1% and 65.4%, respectively, higher than those of the mixtures at the doses of 100 mg/kg of fuziline and 0.5 mg/kg of mesaconitine. In terms of the pain suppression rate, with the increase of the proportion of fuziline, its analgesic effect increases. The dose and medical effect of compound medicines composed of the mixtures in three different proportions have no dose dependence. The mixture of fuziline and mesaconitine in the proportion of 600:1 has the strongest analgesic effect and that in the proportion of 200:1 has the lowest dose and strong effect, reaching the goal of high effect and low toxicity.

Test 6 Analgesic Test of the Mixtures of Fuziline and Hypaconitine in Different Proportions

The test shows the mixtures at the doses of 100 mg/kg of fuziline and 1 mg/kg of hypaconitine have outstanding analgesic effect, with the pain suppression rate of 31.3% and 48.0%, respectively. The mixtures with proportions of fuziline and hypaconitine as 300:1, 100:1 and 100:3 can reduce the reaction of body with the pain suppression rate of 39.1%, 55.3% and 78.4%, respectively. The effect of the mixture in the proportions of 100:1 and 100:3 is stronger than that of 100 mg/kg and that of 1 mg/kg of hypaconitine. In terms of the pain suppression rate, with the increase of the proportion of hypaconitine, its analgesiceffect increases. Therefore, it is deemed that the optimum mixture proportion of fuziline and hypaconitine is 100:3. However, the mixture also involves the highest toxicity, which points out the active synergetic effect between fuziline and hypaconitine on the medical effect and toxicity. In practical use, the dose can be reduced to avoid the toxic reaction under on the premise of no alteration of the mixture proportion of 100:3 between fuziline and hypaconitine.

I. Test Materials

1. Test Drug and Preparation

Fuziline, hypaconitine, near-white powder, supplied by Chengdu Zhizhi Pharmaceuticals Co., Ltd.

Prepare 250mg of fuziline before mixing with 2.5 ml of dehydrated alcohol, followed by ultrasound-assisted dissolution with 42°C water bath; add distilled water up to 25 ml to compound 10 mg/ml of solution composed of fuziline; prepare 2.5 mg/ml of hypaconitine before mixing with 2.5 ml of dehydrated alcohol, followed by ultrasound-assisted dissolution with 42°C water bath; add distilled water up to 25 ml to compound 0.1 mg/ml of solution composed of hypaconitine; prepare solution of different volumes of fuziline and solution of different volumes of mesaconitine into solution in different proportions of fuziline and mesaconitine according to Table 10.

Table 10. Mixture of fuziline and hypaconitine in different proportions

| Group | Sampling dose(ml) | | | Dose (mg/kg) | | proportion |
|---|---|---|---|---|---|---|
| | Fuziline | Hypaconitine | Distilled water | Fuziline | Hypaconitine | |
| ① | 2.5 | -- | 2.5 | 100 | -- | -- |
| ② | -- | 2.5 | 2.5 | -- | 1 | -- |
| ③ | 3.75 | 1.25 | -- | 150 | 0.5 | 300:1 |
| ④ | 2.5 | 2.5 | -- | 100 | 1 | 100:1 |
| ⑤ | 1.25 | 3.75 | -- | 50 | 1.5 | 100:3 |

Test Drug and Preparation

Enteric coated aspirin tablet, white, 25 mg/pill, supplied by Xuzhou Enhua Pharmaceuticals Co., Ltd, batch No.: 20050312; Take 4 pills of enteric coated aspirin tablet; grind and add with distilled water up to 10 ml to compound 10 mg/ml of solution composed of aspirin.

Then blend 1 ml of dehydrated alcohol and 10 ml of distilled water to compound 10% alcohol.

2. Test Animal

Kunming breed mice with equal number of each gender, weighing 18-22 g, supplied by Experimental Animal Center of Sichuan University, first class animal; certificate: Sichuan Animal Administration, Quality No. 10; free intake of water; breeding house temperature: $21\pm2°C$; comparative humidity: 50-60%.

3. Drugs

Glacial acetic acid, analytically pure, Chengdu Chemical Reagent Factory, batch No.: 20050423.

Dehydrated alcohol, analytically pure, Chengdu Kelong Chemical Reagent Factory, batch No.: 20050313.

II. Test Method

Divide randomly the 70 Kunming breed mice, weighing 18-22 g, into 7 groups with equal number of each gender and each group, with the first group as the negative control group and the second group as the positive control group; perfuse the stomach of the first group mice with 0.2 ml/10g of 10% alcohol; perfuse the stomach of the second group mice with 200 mg/kg (20 times the practice dose) of aspirin; the $3^{rd}$, $4^{th}$, $5^{th}$, $6^{th}$ and $7^{th}$ groups are of fuziline, hypaconitine and their three different proportions; perfuse the groups with solution with fuziline, hypaconitine and their three different proportions; the administration volume is 0.2 ml/10g; inject into the stomach cavity of the mice with 0.2 ml of 0.6% glacial acetic acid 30 minutes after administration; observe the number of body twisting of each mouse 5 to 10 minutes after the injection; make statistical analysis of the data to calculate the increasing rate of pain threshold and the standard deviation of each group; compare the intergroup differences between each drug group and the negative control group with t-test.

III. Test Results

The injection of acetic acid causes the mice lasting pain with reactions of frequent hindleg stretching, rump rising (body twisting). The number of body twisting caused by acetic acid in the positive control group of aspirin is declining remarkably ($p<0.001$), with remarkable analgesic effect and the pain suppression rate of 82.7%. The mixtures at the doses of 100 mg/kg of fuziline and 1 mg/kg of hypaconitine can remarkably ease the reaction of body twisting caused by acetic acid, with the pain suppression rate of 31.3% and 48.0% respectively. The mixtures with proportions of fuziline and hypaconitine as 300:1, 100:1 and 100:3 can also ease the body twisting reaction to various degrees. The number of body twisting is quite from that of the negative control group with remarkable analgesic effect and the pain suppression rate of 39.1 %, 55.3% and 78.4%. The test result is as Table 11 shows. In terms of the pain suppression rate, the analgesic effect of the mixture of fuziline and hypaconitine in the proportion of 100:3 is the strongest. Therefore, the optimum proportion of the mixture of fuziline and hypaconitine is 100:3. However, we have also proved that the proportion of 100:3 of the mixture of fuziline and hypaconitine produces the strongest reaction of atrophy and hiccup, with two mice dead 30 minutes after the administration. The data comes from the observations of the remaining 8 mice. Based on our previous acute toxicology of fuziline and hypaconitine, the maximum tolerance dose can be up to 1 g/kg; The $LD_{50}$ of hypaconitine can get to 12.8 g/kg and 95% of the confidence interval can get to 10.93-14.99 mg/kg, with no mice dead at 8.19 mg/kg. Therefore, it is deemed that fuziline and hypaconitine has active synergetic effect not only on medical effect but also on toxicity. Considering its toxicity, in practical use, the dose can be reduced to avoid the toxic reaction on the premise of no alteration of the mixture proportion of 100:3 between fuziline and hypaconitine.

Table 11. Effects of the mixture of fuziline and hypaconitine in different proportions on the pain reaction of mice caused by acetic acid

| Group | Dose (mg/kg) | | Number of animals | Body twisting number ($\bar{x}\pm SD$) | Pain suppression rate (%) |
|---|---|---|---|---|---|
| | Fuziline | Hypaconitine | | | |
| Negative control | -- | -- | 10 | 17.9±6.0 | -- |
| Aspirin | -- | -- | 10 | 3.1±3.3*** | 82.7 |
| ① | 100 | -- | 10 | 12.3±5.8* | 31.3 |
| ② | -- | 1 | 10 | 9.3±7.8* | 48.0 |
| ③ | 150 | 0.5 | 10 | 10.9±7.4* | 39.1 |
| ④ | 100 | 1 | 10 | 8.0±7.9** | 55.3 |
| ⑤ | 50 | 1.5 | 10 | 3.9±5.2*** | 78.4 |
| Note: t-test, compared with that of the negative control group, *p<0.05, **p<0.01, ***p<0.001<br>*Two out of the 10 mice after stomach perfusion of fuziline and hypaconitine of the ⑤ group died after 30 minutes. The data comes from the observation of the remaining 8 mice. | | | | | |

IV. Conclusion

The mixtures at the doses of 100 mg/kg of fuziline and 1 g/kg of hypaconitine have outstanding analgesic effect, with the pain suppression rate of 31.3% and 48.0%, respectively. The mixtures with proportions of fuziline and hypaconitine as 300:1, 100:1 and 100:3 can reduce the reaction of body with the pain suppression rate of 39.1%, 55.3% and 78.4%, respectively. The effect of the mixture in the proportions of 100:1 and 100:3 is stronger than that of 100 mg/kg and that of 1 mg/kg of hypaconitine. In terms of the pain suppression rate, with the increase of the proportion of hypaconitine, its analgesic effect increases. Therefore, it is deemed that the optimum mixture proportion of fuziline and hypaconitine is 100:3. However, the mixture also involves the highest toxicity, which points out the active synergetic effect between fuziline and hypaconitine on the medical effect and toxicity. In practical use, the dose can be reduced to avoid the toxic reaction under on the premise of no alteration of the mixture proportion of 100:3 between fuziline and hypaconitine.

Test 7 Analgesic Test of the Mixtures of Fuziline and Benzoylmesaconitine in Different Proportions

The test shows the mixtures at the doses of 100 mg/kg of fuziline and benzoylmesaconitine have outstanding analgesic effect, with the pain suppression rate of 31.3% and 34.6%, respectively. The mixtures with proportions of fuziline and benzoylmesaconitine as 75:25, 50:50 and 25:75 can reduce the reaction of body with the pain suppression rates of 39.7%, 46.9% and 50.3%, respectively, i.e., higher than those of the 100 mg/kg of fuziline and benzoylmesaconitine. In terms of the pain suppression rate, with the increase of the proportion of benzoylmesaconitine, its analgesic effect increases. The proportion of the mixture of fuziline and benzoylmesaconitine as 25:75 has the strongest analgesic effect among the 3 different proportions. Therefore, it is deemed that the optimum mixture proportion of fuziline and benzoylmesaconitine is 25:75.

I. Test Materials

1. Test Drug and Preparation

Fuziline, near-white powder, benzoylmesaconitine, light yellow powder, supplied by Chengdu Zhizhi Pharmaceuticals Co., Ltd.

Prepare 250mg of fuziline and benzoylmesaconitine each before mixing with 2.5 ml of dehydrated alcohol, followed by ultrasound-assisted dissolution with 42°C water bath; add distilled water up to 25 ml to compound 10 mg/ml of solution composed of fuziline and benzoylmesaconitine each; prepare solution of different volumes of fuziline and solution of different volumes of mesaconitine into solution in different proportions of fuziline and benzoylmesaconitine according to Table 12.

Enteric coated aspirin tablet, white, 25 mg/pill, supplied by Xuzhou Enhua Pharmaceuticals Co., Ltd, batch No.:20050312; Take 4 pills of enteric coated aspirin tablet; grind and add with distilled water up to 10 ml to compound 10 mg/ml of solution composed of aspirin.

Then blend 1 ml of dehydrated alcohol and 10 ml of distilled water to compound 10% alcohol.

Table 12. Mixture of fuziline and benzoylmesaconitine in different proportions

| Group | Dose (ml) | | | Dose (mg/kg) | | Proportion (%) |
|---|---|---|---|---|---|---|
| | Fuziline | Benzoylmes aconitine | Distilled water | Fuziline | Benzoylme saconitine | |
| ① | 2.5 | -- | 2.5 | 100 | -- | -- |
| ② | -- | 2.5 | 2.5 | -- | 100 | -- |
| ③ | 3.75 | 1.25 | -- | 150 | 50 | 75:25 |
| ④ | 2.5 | 2.5 | -- | 100 | 100 | 50:50 |
| ⑤ | 1.25 | 3.75 | -- | 50 | 150 | 25:75 |

2. Test Animal

Kunming breed mice with equal number of each gender, weighing 18-22 g, supplied by Experimental Animal Center of Sichuan University, first class animal; certificate: Sichuan Animal Administration, Quality No. 10; free intake of water; breeding house temperature: 21±2°C; comparative humidity: 50-60%.

3. Drugs

Glacial acetic acid, analytically pure, Chengdu Chemical Reagent Factory, batch No.: 20050423.

Dehydrated alcohol, analytically pure, Chengdu Kelong Chemical Reagent Factory, batch No.: 20050313.

II. Test Method

Divide randomly the 70 Kunming breed mice, weighing 18-22 g, into 7 groups with equal number of each gender and each group, with the first group as the negative control group and the second group as the positive control group; perfuse the stomach of the first group mice with 0.2 ml/10g of 10% alcohol; perfuse the stomach of the second group mice with 200 mg/kg (20 times the practice dose) of aspirin; the 3$^{rd}$, 4$^{th}$, 5$^{th}$, 6$^{th}$ and 7$^{th}$ group are of fuziline, benzoylmesaconitine and their three different proportions; perfuse the groups with solution with fuziline, benzoylmesaconitine and their three different proportions; the administration volume is 0.2 ml/10g; inject into the stomach cavity of the mice with 0.2 ml of 0.6% glacial acetic acid 30 minutes after administration; observe the number of body twisting of each mouse 5 to 10 minutes after the injection; make statistical analysis of the data to calculate the increasing rate of pain threshold and the standard deviation of each group; compare the intergroup differences between each drug group and the negative control group with t-test.

III. Test Results

The injection of acetic acid causes the mice lasting pain with reactions of frequent hindleg stretching, rump rising (body twisting). The number of body twisting caused by acetic acid in the positive control group of aspirin is declining remarkably ($p<0.001$), with remarkable analgesic effect and the pain suppression rate of 82.7%. The mixtures of fuziline and 100 mg/kg of benzoylmesaconitine can remarkably ease the reaction of body twisting caused by acetic acid, with the pain suppression rate of 31.3% and 34.6%, respectively. The mixtures with proportions of fuziline and benzoylmesaconitine as 75:25, 50:50 and 25:75 can also ease the body twisting reaction to various degrees. The number of body twisting is quite from that of the negative control group with remarkable analgesic effect and the pain suppression rate of 39.7%, 46.9% and 50.3%, respectively, higher than those of the 100 kg/kg of fuziline and benzoylmesaconitine as shown in Table 13. In terms of the pain suppression rate, with the increase of the proportion of benzoylmesaconitine, its analgesic effect increases. The proportion of the mixture of fuziline and benzoylmesaconitine as 25:75 has the strongest analgesic effect among the 3 different proportions. Therefore, it is deemed that the optimum mixture proportion of fuziline and benzoylmesaconitine is 25:75.

Table 13. Effects of the mixture of fuziline and benzoylmesaconitine in different proportions on the pain reaction of mice caused by acetic acid

| Group | Dose (mg/kg) | | Number of animals | Body twisting number ($\bar{x}$±SD) | Pain suppression rate (%) |
|---|---|---|---|---|---|
| | Fuziline | Benzoylmesa conitine | | | |
| Negative control | -- | -- | 10 | 17.9±6.0 | -- |
| Aspirin | -- | -- | 10 | 3.1±3.3*** | 82.7 |
| ① | 100 | -- | 10 | 12.3±5.8* | 31.3 |

(continued)

| Group | Dose (mg/kg) | | Number of animals | Body twisting number ($\bar{x}\pm$SD) | Pain suppression rate (%) |
|---|---|---|---|---|---|
| | Fuziline | Benzoylmesaconitine | | | |
| ② | -- | 100 | 10 | 11.7±7.2* | 34.6 |
| ③ | 150 | 50 | 10 | 10.8±8.4* | 39.7 |
| ④ | 100 | 100 | 10 | 9.5±6.7** | 46.9 |
| ⑤ | 50 | 100 | 10 | 8.9±6.1** | 50.3 |
| Note: t-test, compared with that of the negative control group, *p<0.05, **p<0.01, ***p<0.001 | | | | | |

IV. Conclusion

The mixtures at the doses of 100 mg/kg of fuziline and 1 g/kg of benzoylmesaconitine have outstanding analgesic effect, with the pain suppression rate of 31.3% and 34.6.0%, respectively. The mixtures with proportions of fuziline and benzoylmesaconitine as 75:25, 50:50 and 25:75 can reduce the reaction of body with the pain suppression rate of 39.7%, 46.9% and 50.3%, respectively, higher than those of the 100 kg/kg of fuziline and benzoylmesaconitine as shown in Table 13. In terms of the pain suppression rate, with the increase of the proportion of benzoylmesaconitine, its analgesic effect increases. The proportion of the mixture of fuziline and benzoylmesaconitine as 25:75 has the strongest analgesic effect among the 3 different proportions. Therefore, it is deemed that the optimum mixture proportion of fuziline and benzoylmesaconitine is 25:75.

Test 8 Analgesic Test of Intramuscular Injection of Fuziline

The test shows intramuscular injection of fuziline at the dose of 100 and 25mg/kg has outstanding analgesic effect, with the pain suppression rate of 93.9% and 52.0%, respectively. The analgesic effect of intramuscular injection at the same dose is stronger than that of stomach perfusion. The same goes for toxicity. This shows that in practical use, the dose of intramuscular injection of fuziline should be much lower than that of stomach perfusion to avoid a toxic reaction.

I. Test Materials

1. Test Drug and Preparation

Fuziline, near-white powder, supplied by Chengdu Zhizhi Pharmaceuticals Co., Ltd.

Prepare 125mg of fuziline before mixing with 1.25 ml of dehydrated alcohol, followed by ultrasound-assisted dissolution with 42°C water bath; add normal saline up to 25 ml to compound 5 mg/ml of solution composed of fuziline; take 2.5 ml of the prepared solution to be mixed with normal saline to 10 ml to compound 1.25 mg/ml solution.

Enteric coated aspirin tablet, white, 25 mg/pill, supplied by Xuzhou Enhua Pharmaceuticals Co., Ltd, batch No.: 20050312; take 4 pills of enteric coated aspirin tablet; grind and add with distilled water up to 10 ml to compound 10mg/ml of solution composed of aspirin.

Then blend 0.5 ml of dehydrated alcohol and 10 ml of distilled water to compound 5% of alcohol.

2. Test Animal

Kunming breed mice with equal number of each gender, weighing 18-22 g, supplied by Experimental Animal Center of Sichuan University, first class animal; certificate: Sichuan Animal Administration, Quality No. 10; free intake of water; breeding house temperature: 21±2°C; comparative humidity: 50-60%.

3. Drugs

Glacial acetic acid, analytically pure, Chengdu Chemical Reagent Factory, batch No.: 20050423.

Dehydrated alcohol, analytically pure, Chengdu Kelong Chemical Reagent Factory, batch No.: 20050313.

II. Test Method

Divide randomly the 50 Kunming breed mice, weighing 18-22 g, into 5 groups with equal number of each gender and each group, with the first group as the negative control group and the second group as the positive control group; inject 0.2 ml/10g of 5% alcohol into the hindleg muscle of the first group mice; perfuse the stomach of the second group mice with 200 mg/kg (20 times the practice dose) of aspirin; perfuse the stomach of the third group mice with 5 mg/kg of fuziline solution; inject with 5 mg/kg of fuziline solution into the hindleg muscle of the fourth group mice; inject with 1.25 mg/kg of fuziline solution into the hindleg muscle of the fifth group mice; each administration volume is 0.2 ml/10g; inject into the stomach cavity of each mice with 0.2 ml of 0.6% glacial acetic acid 30 minutes after administration; observe the number of body twisting of each mouse 5 to 10 minutes after the injection; make statistical analysis of the data to calculate the increasing rate of pain threshold and the standard deviation of each group; compare the intergroup differences between each drug group and the negative control group with t-test.

III. Test Results

The injection of acetic acid causes the mice lasting pain with reactions of frequent hindleg stretching, rump rising (body twisting). The number of body twisting caused by acetic acid in the positive control group of aspirin is declining remarkably ($p<0.001$), with remarkable analgesic effect and the pain suppression rate of 81.6%. Intramuscular injection of fuziline and stomach perfusion of 100 mg/kg of fuziline can both remarkably ease the reaction of body twisting caused by acetic acid, with the pain suppression rate of 93.9% and 36.1 %, respectively. However, the analgesic effect of intramuscular injection is far stronger than that of stomach perfusion. Even the analgesic effect of intramuscular injection at the dose of 25 mg/kg is stronger than that of stomach perfusion at the dose of 100 mg/kg, whose pain suppression rate is 52.0%, as Table 14 shows. We have also proved that intramuscular injection of fuziline at the dose of 100 mg/kg produces the apparent reaction of atrophy and hiccup. This example shows that in practical use, the dose of intramuscular injection of fuziline should be much lower than that of stomach perfusion to avoid the toxic reaction.

Table 14. Effects of fuziline on the pain reaction of mice caused by acetic acid

| Group | Dose (mg/kg) | Administration channel | Number of animals | Body twisting number ($\bar{x}\pm SD$) | Pain suppression rate (%) |
|---|---|---|---|---|---|
| Negative control | -- | im. | 10 | 27.7±12.4 | -- |
| aspirin | 200 | ig. | 10 | 5.1±6.0*** | 81.6 |
| fuziline | 100 | ig. | 10 | 17.7±7.4* | 36.1 |
| | 100 | im. | 10 | 1.7±2.6*** | 93.9 |
| | 25 | im. | 10 | 13.3±16.0* | 52.0 |
| Note: t-test, compared with that of the negative control group, *p<0.05, **p<0.01, ***p<0.001 | | | | | |

IV. Conclusion

Intramuscular injection of fuziline at the dose of 100 and 25 mg/kg has outstanding analgesic effect, with the pain suppression rate of 93.9% and 52.0%, respectively. The analgesic effect of intramuscular injection at the same dose is stronger than that of stomach perfusion. The same goes for toxicity. It proves that in practical use, the dose of intramuscular injection of fuziline should be much lower than that of stomach perfusion to avoid a toxic reaction.

Test 9. Analgesic Test of Skin Administration of Fuziline

The test shows skin administration of fuziline at the dose of 800 mg/kg can remarkably ease the reaction of body twisting caused by acetic acid, with the pain suppression rate 44.4%. Though the pain suppression rate is 36.1% at the dose of 400 mg/kg, the number of body twisting is not apparently different from that of the negative control group, there being no remarkable analgesic effect. It indicates that the skin absorption of fuziline is not quite effective. If the addition of excipient and the skin absorption of fuziline can be improved, the dose as well as the cost can be reduced.

I. Test Materials

1. Test Drug and Preparation

Fuziline, near-white powder, supplied by Chengdu Zhizhi Pharmaceuticals Co., Ltd.

Prepare 400mg of fuziline before dissolved with 2.5 ml of ether; then immerse with 2.1 g of medicinal Vaseline evenly; dry the solution in the bake oven of 37°C for a night to evaporate ether before made into Vaseline plaster composed of approximate 16mg/g of fuziline.

Enteric coated aspirin tablet, white, 25 mg/pill, supplied by Xuzhou Enhua Pharmaceuticals Co., Ltd, batch No.:20050312; Take 4 pills of enteric coated aspirin tablet; grind and add with distilled water up to 10 ml to compound 10 mg/ml of solution composed of aspirin;

Take 2g of medicinal Vaseline mixed with 2.5 ml of ether and blend evenly; dry the solution in the bake oven of 37°C for a night to evaporate ether before made into Vaseline plaster for the negative control group.

2. Test Animal

Kunming breed mice with equal number of each gender, weighing 18-22 g, supplied by Experimental Animal Center of Sichuan University, first class animal; certificate: Sichuan Animal Administration, Quality No. 10; free intake of water; breeding house temperature: 21±2°C; comparative humidity: 50-60%.

3. Drugs

Ether, analytically pure, Chengdu Kelong Chemical Reagent Factory, batch No.: 20050407.

Sodium sulfide, analytically pure, Chengdu Chemical Reagent Factory, batch No.: 20050921.

Glacial acetic acid, analytically pure, Chengdu Kelong Chemical Reagent Factory, batch No.: 20050423.

Medicinal Vaseline, Wuhan Petrochemical Plant.

II. Test Method

Divide randomly the 40 Kunming breed mice, weighing 18-22 g, into 4 groups with equal number of each gender and each group. The day before administration, cut off the hair on the back of the mice with scissor and rid an area (1.5cm×1.5cm) of hair with 8% of sodium sulfide. The first group is the negative control group. Smear an even layer of Vaseline plaster 0.1g/piece onto the hair-disposed area of the of negative control group mice, covered lightly with a piece of gauze (1.5cm×1.5cm). The second group, as the positive control group is given stomach perfusion with 200 mg/kg of aspirin (20 times the practice use). Smear an even layer of 0.1 g of plaster composed of 16 mg/g of fuziline onto the hair-disposed area of each of the third group mice; Smear an even layer of 0.05 g of plaster composed of 16 mg/g of fuziline onto the hair-disposed area of each of the fourth group mice; both are covered lightly with a piece of gauze (1.5cm×1.5cm). Except that the mice of the second group are injected into the stomach cavity with 0.6% glacial acetic acid solution 30 minutes after the smearing, the mice of all other groups are injected into the stomach cavity with 0.6% glacial acetic acid solution 1.5 hours after the smearing; observe the number of body twisting of each mouse 5 to 10 minutes after the injection; make statistical analysis of the data to calculate the increasing rate of pain threshold and the standard deviation of each group; compare the intergroup differences between each drug group and the negative control group with t-test.

$$\text{Pain suppression rate (\%)} = \frac{\text{Body twisting number of blank group} - \text{Body twisting number of group administered drugs}}{\text{Body twisting number of blank group}} \times 100\%$$

III. Test Result

The injection of acetic acid causes the mice lasting pain with reactions of frequent hindleg stretching, rump rising (body twisting). The number of body twisting caused by acetic acid in the positive control group of aspirin is declining remarkably (p<0.001), with remarkable analgesic effect and the pain suppression rate of 81.6%. Skin administration of fuziline at the dose of 800 mg/kg can remarkably ease the reaction of body twisting caused by acetic acid, with the pain suppression rate 44.4%. Though the pain suppression rate is 36.1% at the dose of 400 mg/kg, the number of body twisting is not apparently from that of the negative control group, there being no remarkable analgesic effect, as shown in Table 15. This indicates that the skin absorption of fuziline is not quite effective. If the addition of excipient and the skin absorption of fuziline can be improved, the dose as well as the cost can be reduced.

Table 15 Effects of the external use of fuziline on the pain reaction of mice caused by acetic acid

| Group | Dose (mg/kg) | Administration channel | Number of animals | Body twisting number ($\bar{x}$±SD) | Pain suppression rate (%) |
|---|---|---|---|---|---|
| Negative control | -- | external | 10 | 27.7±12.4 | -- |
| Aspirin | 200 | ig. | 10 | 5.1±6.0*** | 81.6 |
| Fuziline | about 800 | external | 10 | 15.4±8.9* | 44.4 |
| | about 400 | | 10 | 17.7±8.8 | 36.1 |
| Note: t-test, compared with that of the negative control group, *p<0.05, **p<0.01, ***p<0.001 | | | | | |

IV. Conclusion

Skin administration of fuziline at the dose of 800 mg/kg can remarkably ease the reaction of body twisting caused by acetic acid, with the pain suppression rate 44.4%. Though the pain suppression rate is 36.1% at the dose of 400 mg/kg, the number of body twisting is not apparently from that of the negative control group, there being no remarkable analgesic effect. It indicates that the dose of fuziline is much higher when used externally on skin, which means the skin

absorption of fuziline is not quite effective. If the addition of excipient and the skin absorption of fuziline can be improved, the dose as well as the cost can be reduced.

In conclusion, the compatibility of fuziline and other aconite alkaloids is within the safe range of dose. The single use of fuziline acts effectively with low toxicity and high safety. The compatibility of fuziline and the other three aconite alkaloids can bring about synergistic effect, what's more, the drug mixtures and their effect after compatibility is not absolutely in the simple linear relation of dose and effect, i.e. the larger dose, the better effect, but the compositions have the best effect when the active ingredients are in a specific proportion. The drug mixture in the optimum proportion exerts best effect with apparent decline of toxicity and the least dose, achieving the goals of significant effect and low toxicity, providing a new option for the clinic practice.

## Claims

1. A pharmaceutical composition having analgesic effect comprising auxiliary materials applicable to the preparations, and drug compounds as active medical ingredients provided in the following weight proportion: 200-600 parts of fuziline, 0-600 parts of Benzoylmesaconitine, 0-3 parts of Mesaconitine and 0-6 parts of Hypaconitine of Formula (I),

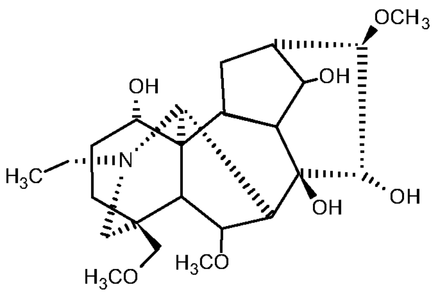

**(I)**

wherein the combined weight proportion of Benzoylmesaconitine, Mesaconitine and Hypaconitine shall not be zero.

2. The pharmaceutical composition of Claim 1, wherein the active medical ingredients are provided in the following weight proportions: 200-600 parts of fuziline, 200-600 parts of Benzoylmesaconitine, 1-3 parts of Mesaconitine, and 2-6 parts of Hypaconitine.

3. The pharmaceutical composition of Claim 2, wherein the active medical ingredients are provided in the following weight proportions: 200 parts of fuziline, 200 parts of Benzoylmesaconitine, 3 parts of Mesaconitine, and 6 parts of Hypaconitine.

4. The pharmaceutical composition of Claim 1, wherein the active medical ingredients are provided in the following weight proportions: 200-600 parts of fuziline and 1-3 parts of Mesaconitine.

5. The pharmaceutical composition of Claim 1, wherein the active medical ingredients are provided in the following weight proportions: 600 parts of fuziline and 1 part of Mesaconitine.

6. The pharmaceutical composition of Claim 1, wherein the active medical ingredients are provided in the following weight proportions: 100-300 parts of fuziline and 1-3 parts of Hypaconitine.

7. The pharmaceutical composition of Claim 6, wherein the active medical ingredients are provided in the following weight proportions: 100 parts of fuziline and 3 parts of Hypaconitine.

8. The pharmaceutical composition of Claim 1, wherein the active medical ingredients are provided in the following weight proportions: 25-75 parts of fuziline and 25-75 parts of Benzoylmesaconitine.

9. The pharmaceutical composition of Claim 8, wherein the active medical ingredients are provided in the following weight proportions: 25 parts of fuziline and 75 parts of Benzoylmesaconitine.

**10.** The pharmaceutical composition of any of claims 1-9 formulated for oral, external, or injectable use.

**11.** The pharmaceutical composition of claim 10 formulated for oral use in form of an oral liquid, a troche, a capsule, a granule, or a drop pill.

**12.** The pharmaceutical composition of claim 10 formulated for external use in form of a medicinal dressing, an ointment, a suppository, or a lotion.

# INTERNATIONAL SEARCH REPORT

International application No.
PCT/CN 2005/001384

A. CLASSIFICATION OF SUBJECT MATTER

A61K 31/439, A61P 25/04, A61P 29/02

According to International Patent Classification (IPC) or to both national classification and IPC

B. FIELDS SEARCHED

Minimum documentation searched (classification system followed by classification symbols)

IPC7: A61K, A61P

Documentation searched other than minimum documentation to the extent that such documents are included in the fields searched

Cprs, CNKI Full-Text database, Chinese Medicine Abstract

Electronic data base consulted during the international search (name of data base and, where practicable, search terms used)

WPI, EPODOC, PAJ, Medline, CA; fuzi, analgesic, fuziline, hypaconitine, mesaconitine, benzoylmesacotine, neoline, hydroxyneoline

C. DOCUMENTS CONSIDERED TO BE RELEVANT

| Category* | Citation of document, with indication, where appropriate, of the relevant passages | Relevant to claim No. |
|---|---|---|
| X | CN 1147766A(SUZUKI F) 16. Apr 1997, see despcriptions, page 2, line 6-10, page 7, page 25, line 10-page 26, line 24, page 28, example 6 | 1,10-12 |
| X | Wang Xiakai et al. Alkaloids of cultivated *Aconitum carmichaeli* II. CHIN PHARM J, 1996 February, Vol. 31 No. 2: 74-77 | 1,10-12 |
| X | Han Gongyu, et al. Stedies on the alkaloids and a new cardiac principle isolated from jiangyou fuzi(*Aconitum Carmichaeli Deb.x*). NATURAL PRODUCT RESEARCH AND DEVELOPMENT, 1997 Sep, vol. 9 No. 3: 30-34 | 1,10-12 |
| X | Chen Hong-chao, et al. Chemical constituents from Acotinum carmichaeli. NATURAL PRODUCT RESEARCH AND DEVELOPMENT, 2003 vol. 15 No. 4: 324-325, 340 | 1,10-12 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 22. Nov 2005(22.11.2005) | 08 · DEC 2005 (08 · 12 · 2005) |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer LEI, Yaolong<br>Telephone No. (86-10)62085254 |

Form PCT/ISA /210 (second sheet) (April 2005)

# INTERNATIONAL SEARCH REPORT

International application No.
PCT/CN2005/001384

C (Continuation). DOCUMENTS CONSIDERED TO BE RELEVANT

| Category* | Citation of document, with indication, where appropriate, of the relevant passages | Relevant to claim No. |
|---|---|---|
| X | CN 1147766A(SUZUKI F) 16. Apr 1997, see despcriptions page 2, line 6-10, page 7, page 25, line 10-page 26, line 24, page 28, example 6 | 2-9 |

Form PCT/ISA /210 (continuation of second sheet ) (April 2005)

**INTERNATIONAL SEARCH REPORT**
Information on patent family members

International application No.
PCT/CN2005/001384

| Patent Documents referred in the Report | Publication Date | Patent Family | Publication Date |
|---|---|---|---|
| CN 1147766 A | 16. 04. 1997 | CA2185825 A1 | 28. 09. 1995 |
| | | WO9525517 A1 | 28. 09. 1995 |
| | | AU1960595 A | 09. 10. 1995 |
| | | JP7524536 T | 24. 09. 1996 |
| | | EP0750908 A1 | 02. 01. 1997 |
| | | KR97701543 A | 12. 04. 1997 |
| | | CN1147766 A | 16. 04. 1997 |
| | | AU689235 B2 | 26.03. 1998 |
| | | US5908857 A | 01. 06. 1999 |
| | | US6030980 A | 29. 02. 2000 |

Form PCT/ISA /210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHANG DIHUA.** *Chinese Traditional and Herbal Medicine,* 1982, vol. 73 (11), 1-4
- **ZHANG YONGZHONG.** *Chinese Traditional and Herbal Medicine,* 2002, vol. 33 (2), 106-109
- **CHEN HONGCHAO.** Chemical Constituents from Aconitum Carmichaeli. *Natural Product Research and Development,* 2003, vol. 15 (4
- **OU MING.** Handbook of Brachylogy Component of Chinese Traditional Drugs. 203-204